# EUROPEAN PATENT SPECIFICATION

(11) **EP 0 642 642 B1**
(45) Date of publication and mention of the grant of the patent: **27.03.1996**
(21) Application number: 92923247.8
(22) Date of filing: 11.11.1992
(51) Int. Cl.: F17C 13/00, A61M 16/10, B67D 5/33

(54) **CONNECTION TO A GAS CYLINDER**
ANSCHLUSSSTÜCK FÜR EINE GASFLASCHE
DISPOSITIF DE RACCORDEMENT A UN CYLINDRE DE GAZ

(30) Priority: 14.11.1991 GB 9124195
(43) Date of publication of application: 15.03.1995
(73) Proprietor: THE BOC GROUP PLC, Windlesham, Surrey GU20 6HT (GB)
(72) Inventor: BRAATZ, Robert, Edwin, Sun Prairie, WI 53591 (US); SANSOM, Gordon, George 2 Laurel Grove, Keighley BD20 0AY (GB)
(74) Representative: Gough, Peter
(86) International application number: GB9202088
(87) International publication number: WO9310392

(56) References cited:
- EP-A- 0 338 518
- WO-A-86/04175
- CH-A- 468 045
- DE-U- 8 716 413
- FR-A- 2 502 134
- US-A- 4 015 865
- US-A- 4 302 980
- US-A- 4 521 676

## Description

The present invention relates to techniques for forming connections to gas cylinders, and to an adaptor or mounting block for use in forming such connections.

Anaesthetic agents are commonly administered to a patient by means of vaporiser suspended in a carrier gas, which might be for example oxygen, nitrogen, nitrous oxide or a mixture of two or more gases. For adequate control of the administration of drug to a patient, it is essential that, not only the drug, but also the carrier gas be positively and accurately identified to ensure that, amongst other things, the rate of flow of carrier gas can be accurately controlled.

It is known to provide the yoke on the anaesthetic delivery apparatus with formations specific to a particular gas which can mate with corresponding formations on a cylinder containing that gas. The formations on the yoke and the cylinder commonly take the form of cooperating pins and corresponding sockets or recesses respectively. The formations can prevent an incorrect cylinder from being fitted to the yoke.

The use of a series of mating formations on the yoke and the cylinder to identify the gas contained within the cylinder has been found to provide the necessary ability to identify accurately the gas within the cylinder. However, in order for anaesthetic delivery apparatus to be capable of taking a range of gases from respective cylinders, it is necessary for the yokes on which the cylinders are mounted on the apparatus to have formations for mating with each different cylinder type. This can increase the space requirement of the yokes on the apparatus to an unacceptable level. Furthermore, it requires appropriate components to be provided, calibrated for each specific gas, to allow flow of a selected carrier gas from its respective yoke into the delivery apparatus for use.

The present invention provides a technique for connecting a gas cylinder to anaesthetic delivery apparatus which makes use of machine readable indicia associated with the cylinder containing the gas to ensure that a correct cylinder is connected to the apparatus.

Accordingly, in one aspect, the invention provides anaesthetic delivery apparatus which comprises:
(a) a gas cylinder mounting yoke;
(b) means for reading machine readable indicia;
(c) a gas cylinder which bears formations according to the gas contained within the cylinder; and
(d) an adaptor block which can be fitted to the yoke, which has formations corresponding to the formations on the gas cylinder to allow the block and the cylinder to be connected to one another; and
(e) machine readable indicia associated with one of the cylinder and the block, by which the gas cylinder with its associated block can be identified by the delivery apparatus when fitted to the yoke.

The invention enables a cylinder to be mounted on the yoke of anaesthetic delivery apparatus.

The apparatus will preferably include means for measuring the flow rate of gas supplied from the gas cylinder. The provision of means for identifying the gas which is being supplied to the apparatus (in the form of the machine readable indicia) allows the flow rate measuring means to be calibrated by the apparatus automatically according to the gas which is being supplied; it is not necessary for the operator of the apparatus to identify the gas which is being supplied and to set up the apparatus accordingly. A further advantage of the apparatus of the invention is that only one path for flow of gas from the cylinder is required, which can be used for a variety of gases which can be supplied to the apparatus. This is to be contrasted with earlier devices in which separate paths from the gas cylinders were required, each calibrated for the gas in question.

The use of an adaptor block in the connection technique of the invention has the advantage that the yoke provided on the anaesthetic delivery apparatus on which a gas cylinder is to be mounted can be arranged to receive cylinders containing a range of gases, each gas cylinder having formations on it to identify the gas uniquely. The yoke can be changed to receive a cylinder containing a different gas simply by replacement on the yoke of the adaptor block. Appropriate recalibration of the apparatus for other gases can take place following identification of the gas by means of the machine readable indicia.

In addition to reducing the space requirement of the gas cylinder mounting components of anaesthetic apparatus, the technique of the present invention has the further advantage of reducing significantly the complexity of the apparatus while allowing the apparatus to receive a number of different cylinders containing different gases. Thus it is possible for the apparatus to receive a number of different cylinders without the need for a series of valves and associated passageways within the apparatus leading from the multiple cylinder yokes used previously.

The adaptor block may be provided with a single set of formations and machine readable indicia so that it can be used with just a single type of gas cylinder to identify the gas within that cylinder. Preferably, however, the adaptor block is provided with more than one set of formations and indicia so that it can be used with cylinders containing different respective gases. For example, two or more sets of formations may be provided side by side along the block, each set of formations mating with corresponding formations on cylinders of specific gases. Such an adaptor block can be used on different gases simply by selecting the point at which it is fitted to the yoke. Alternatively, or in addition, formations may be provided on opposite sides of an adaptor block so that the block can be arranged to receive different cylinders simply by reversing the orientation of the block on the yoke. This construction has the advantage that the space requirement of the adaptor block is minimised.

The formations on the adaptor block will be selected to mate with the corresponding formations on the gas cylinder. A practice of using a system of pins and corresponding recesses for identifying gas cylinders is well established, and the adaptor block may be provided with formations of this type. However, other types of formations may, of course, be used.

The machine readable indicia, which may be provided on the cylinder or on the adaptor block provided for association with the cylinder, may be readable for example mechanically, optically or magnetically. For example, the indicia might be read mechanically when in the form of appropriate formations such as spring loaded pins which can be received in corresponding recesses, or in the form of recesses for receiving such pins on the cylinder or block. Such indicia can function also as the formations which allow the cylinder or block to be connected to the apparatus.

Indicia may be optically encoded; for example, an optically reflective or absorbing material may be provided in a prearranged pattern on the cylinder or adaptor block if present which allows the cylinder to be identified by means of an appropriate light source and detector. For example, in a preferred construction, the cylinder or block may be provided with a pre-determined pattern of material which ensures that light reflected by the cylinder or block has a pattern characteristic of the cylinder (or of that particular face of the block). For example, a series of dots of a non-reflective material may be provided on the cylinder or adaptor block, the pattern of dots being characteristic of the cylinder or block or of the face of the block. This result might be achieved by providing a black material such as a paint on the cylinder or block, preferably within a recess to minimise damage thereto. The indicia reading means might then take the form of a combination of a light source and a detector for the reflected light.

Other forms of indicia on the cylinder or adaptor block include magnetic indicia, for example provided by a magnetically detectable material in a pre-determined pattern on the cylinder or block.

Preferably, the indicia provided on the cylinder or adaptor block are arranged so that indicia must be identified by the reading means on the delivery apparatus (indicating to the operator that an cylinder or adaptor block is present on the yoke) in order for the delivery apparatus to operate. For example, in the case of optically detected indicia, it is preferred that they be reflective indicia (so that in the absence of a cylinder or block no light is reflected) rather than transmissive indicia (where the absence of a cylinder or block might not be accurately identified by the reading means in the event of equipment failure). This has the advantage of providing an added level of security in the delivery apparatus, indicating to the operator when no gas cylinder or adaptor block is fitted.

Preferably, in addition to the indicia which are read by the reading means on the delivery apparatus, additional indicia are provided on the adaptor block which can be read by the operator of the delivery apparatus to indicate the cylinder which can be fitted to the adaptor block. For example, the name or an appropriate symbol for the relevant gas might be written on the block. When the block is provided with more than one set of formations and machine readable indicia, more than one set of operator readable indicia will generally also be provided.

In a preferred embodiment, the yoke will comprise a spigot, and the adaptor block will have a passageway extending through it in which the spigot can be received when the block is mounted on the yoke. The spigot will generally have a passageway extending through it so that gas supplied from the cylinder through the passageway in the adaptor block can enter the delivery apparatus through the passageway in the spigot on the yoke. When the adaptor block has more than one set of formations and machine readable indicia for forming connections between the anaesthetic delivery apparatus and cylinders containing different gases, the block may be moved between its positions for connection to the different cylinders containing the respective gases by removal from the spigot, and subsequent replacement on the spigot in a different orientation. In another configuration, the block may be moved between its positions for connection to the different gas cylinders by sliding, for example by rotation in the manner of a dial, or by linear movement.

Preferably, means will be provided for retaining the adaptor block on the yoke. For example, this may take the form of a spring loaded ball on one of the yoke and the adaptor block, which fits in an appropriate recess on the other of the yoke and block.

Embodiments of the present invention will now be described by way of example with reference to the accompanying drawings, in which,
Figure 1 is an isometric view of components of the anaesthetic delivery apparatus of the present invention;
Figure 2 is a cross-section through the delivery apparatus shown in Figure 1;
Figure 3 is a side elevation of a first embodiment of the adaptor block;
Figure 4 is an isometric view of a second embodiment of adaptor block;
Figure 5 is a cross-section through anaesthetic delivery apparatus which includes a third embodiment of adaptor block;
Figure 6 is an end elevation of a fourth embodiment of adaptor block;
Figures 7 (a) and (b) are side (partially in section) and end views respectively of a yoke for a gas cylinder with an associated adaptor block;
Figures 8 (a), (b) and (c) are side (partially in section), plan (partially in section), and end views of a yoke to which an adaptor block is fitted; and
Figure 9 is an isometric view of a yoke of anaesthetic delivery apparatus.

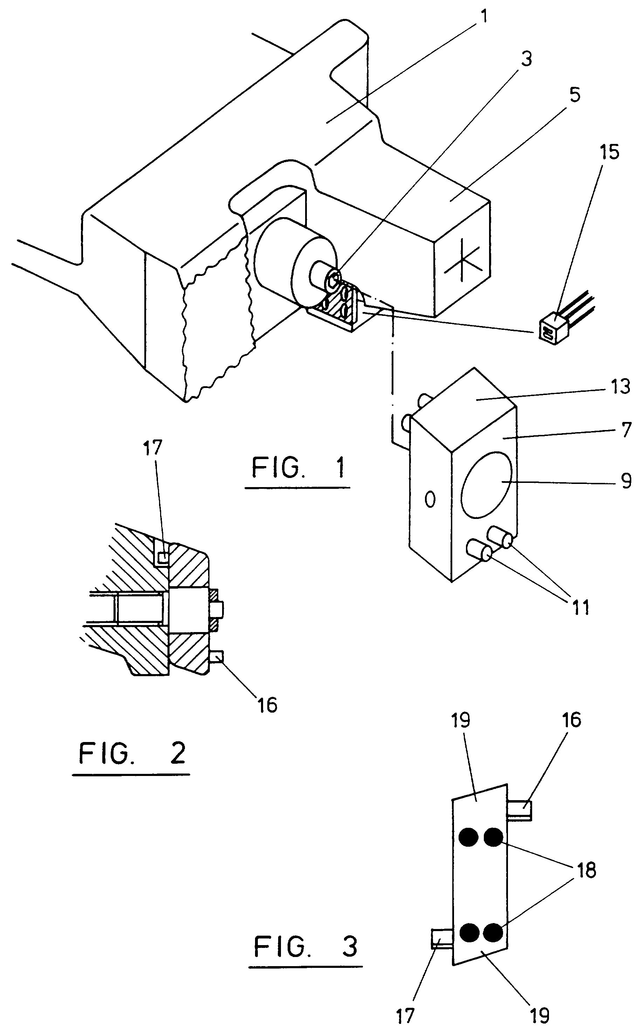

Referring to the drawings, Figure 1 shows anaesthetic delivery apparatus which includes a yoke 1 having a hollow spigot 3 extending from it. Carrier gas passes from the cylinder into the yoke through the opening in the spigot 3. The spigot extends between two guide arms 5 of which just one is shown.

An adaptor block 7 has an opening 9 extending through it in which the spigot 3 is received. The adaptor block is provided with a number of features which are specific to a gas in a cylinder which is to be connected to the block. These features include a pair of identification pins 11 which can be received in corresponding recesses on the cylinder. The block further includes machine readable indicia (which cannot be seen in the view shown in Figure 1) and the chemical formula of the gas which can be supplied through the adaptor block.

The delivery apparatus further includes means for reading the indicia on the adaptor block, which comprises a light source and a series of optical detectors 15, mounted on the guide arm 5 adjacent to the spigot 3. The detectors 15 detect light reflected by the indicia on the adaptor block from the light source.

The adaptor block shown in Figures 1 to 3 has two series of features which allow the block to form connections between the yoke and cylinders containing either of two gases, by appropriate selection of the orientation of the block on the yoke. Each set of features comprises pins, machine readable indicia, and a label for the operator of the apparatus to identify the gas in question.

Figure 2 shows the apparatus shown in Figure 1 once it has been assembled. As can be seen, a first set 16 of the pins 11 is exposed on the face of the adaptor block 7 on which a gas cylinder is received. The second set 17 of pins is on the face of the adaptor block remote from the cylinder.

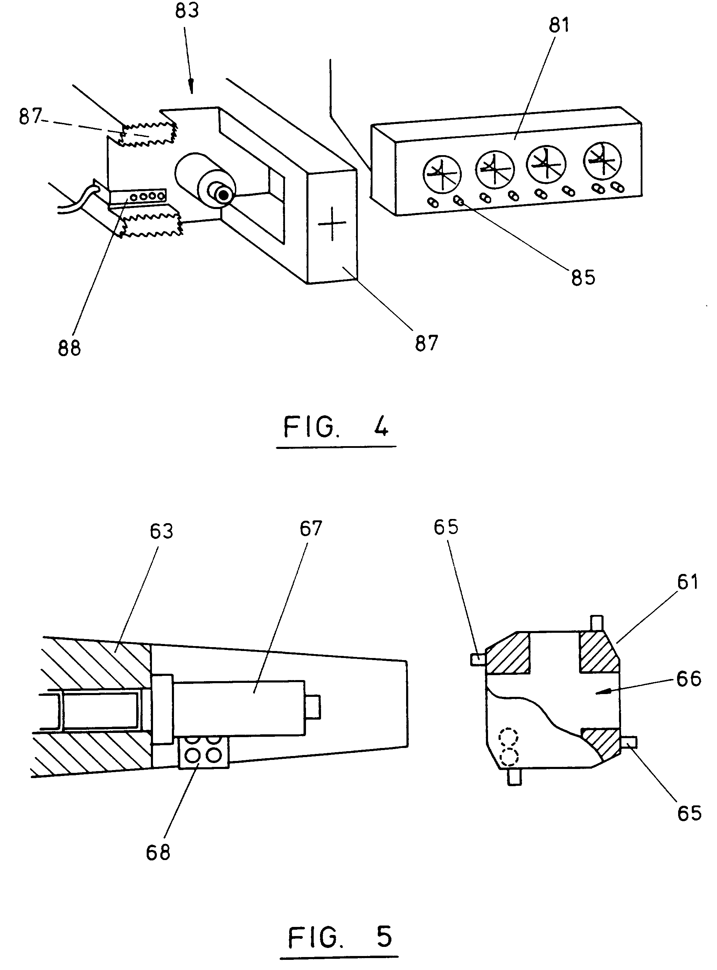

Figure 3 is a side view of the adaptor block shown in Figure 1 from the side hidden from view in Figure 1 as can be seen in Figure 3, the adaptor block 7 has two sets 16, 17 of pins. The block is provided with two sets of machine readable indicia 18, each set of indicia corresponding to a set of the pins 11. Opposite surfaces 19 of the block bear respective labels for the gases in question. The selection of the features on the adaptor block for forming a connection between the yoke and a gas cylinder can be made by rotation of the adaptor block about an axis extending perpendicular to the plane of the page of drawings.

Figure 4 shows an adaptor block 81 which can be used to form connections between a yoke 83 and four different gas cylinders, by appropriate selection of the position of the block on the yoke. To this end, the block is provided with four sets of features including pins 85. Also included are labels and machine readable indicia. The block 83 is moved relative to the yoke 81 by a sliding movement, along a path defined by bridge sections 87. A device 88 is provided on the yoke for reading indicia on the block 81 associated with each set of connection pins 85.

Figure 5 shows an adaptor block 61 which can be used to form connections between a yoke 63 and four different gas cylinders, by appropriate selection of the orientation of the block on the yoke. To this end, the block 61 is provided with four sets of features including pins 65 and associated bores 66 in which a spigot on the yoke can be received. Also included are labels and machine readable indicia 66. The orientation of the block 61 on the spigot 67 is altered by removal from the yoke and appropriate rotation. A device 68 is provided on the yoke for reading indicia on the block 61 associated with each set of connection pins 65.

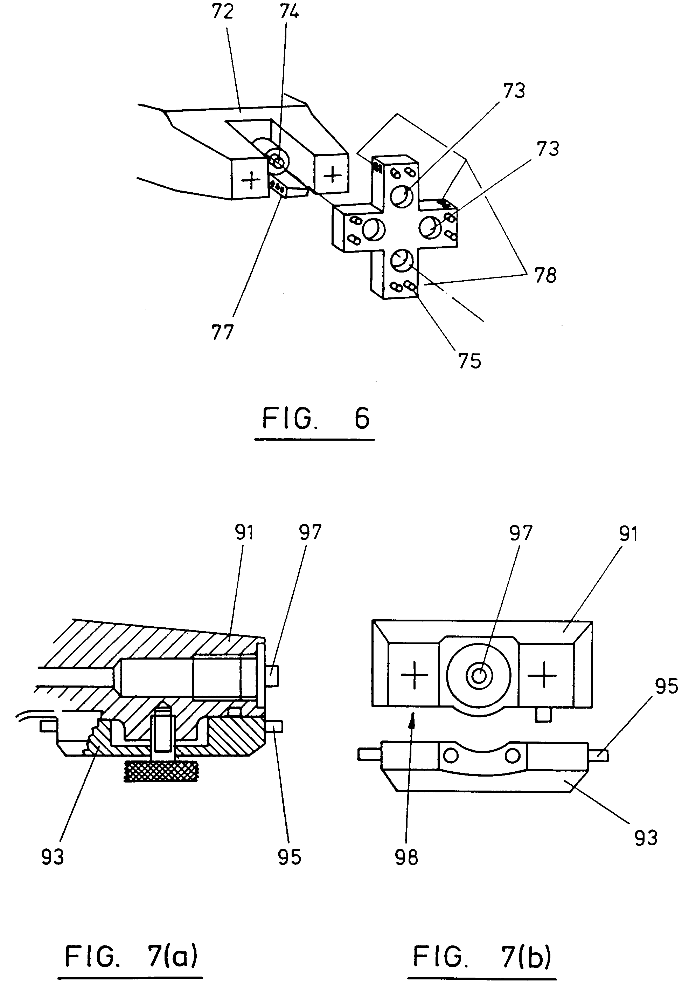

Figure 6 shows another construction of adaptor block 71 which can be used to form connections between a yoke 72 and four different gas cylinders by appropriate selection of the orientation of the block on the yoke. The block has four openings 73 through which a spigot 74 on the yoke can extend, and four sets of pins 75 which can be received in appropriate recesses on respective gas cylinders. The orientation of the block 71 is altered by removal from the yoke and appropriate rotation. A device 77 is provided on the yoke for reading indicia 78 on the block 71 associated with each set of connection pins 75.

Figure 7 shows a yoke 91 which has an adaptor block 93 mounted on a side face. The block has pins 95 extending from its faces, mounted so that the block can be mounted on the yoke with pins on one of the surfaces of the block adjacent to a spigot 97 on which a gas cylinder can be mounted. The pins on the block are arranged for mating with respective cylinders of gases when mounted on the spigot, and the yoke can be adapted for use with a selected gas by selection of the orientation of the block on the yoke. The block bears machine readable indicia and appropriate labels, and the yoke bears a device 98 for reading the indicia on the block.

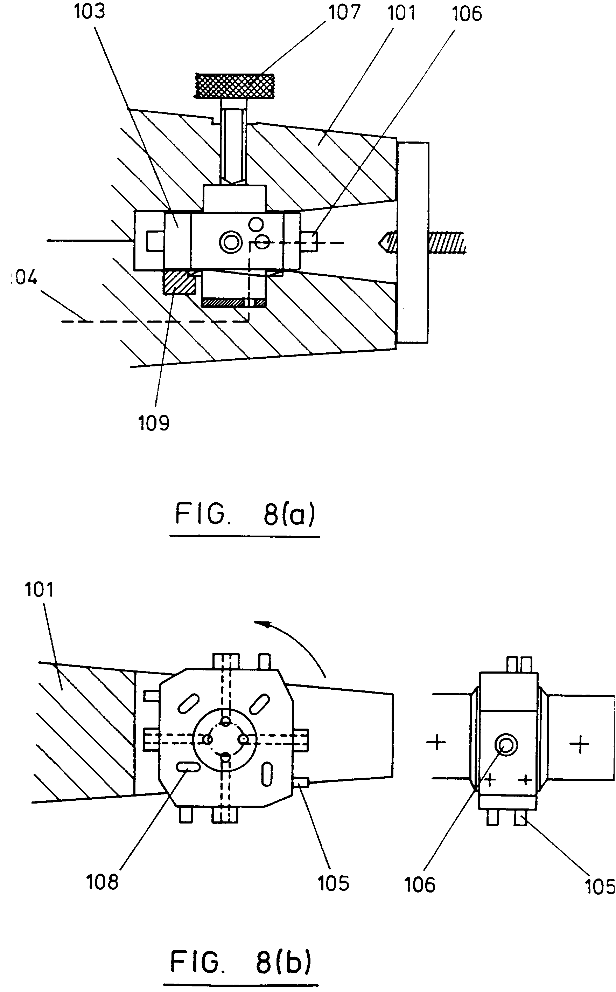

Figure 8 shows a yoke 101 which has an adaptor block 103 permanently and rotatably mounted on it. Gas received from a cylinder passes into the block and into the apparatus for mixture with an anaesthetic agent through the hub on which the block rotates, as shown by the dotted line 104. The block has pins 105 extending from its faces, and a bore 106 associated with each pin for connection to the gas outlet on a gas cylinder. The pins on faces of the block are arranged for mating with respective cylinders of gases, and the yoke can be adapted for use with a selected gas by selection of the orientation of the block on the yoke. Orientation of the block is selected by making loose the clamp 107 to allow the block to be rotated, rotating the block so that the desired face with its pin(s) is presented to a cylinder to be connected to the yoke, and then retightening the clamp 107. The block bears machine readable indicia 108 and appropriate labels, and the yoke bears a device 109 for reading the indicia on the block.

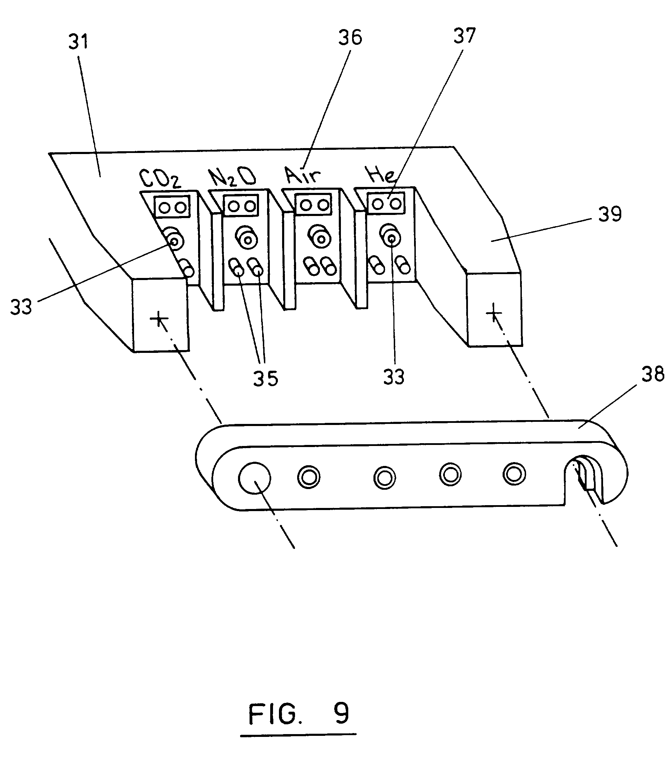

Figure 9 shows a yoke 31 which has mounted on it an adaptor block with four sets of features which allow it to form a connection to cylinders of respective specified gases. For each spigot 33, there are provided pins 35 which can be received in appropriate recesses in the gas cylinder. Also provided are labels 36 for the gases in question and machine readable indicia 37.

Once a gas cylinder has been connected to the pins 35 and spigot 33, a clamping bar 38 is connected to the arms 39 to hold the cylinder in engagement with the spigot.

## Claims

1. Anaesthetic delivery apparatus which comprises:
(a) a gas cylinder mounting yoke (1);
(b) means (15) for reading machine readable indicia;
(c) a gas cylinder which bears formations according to the gas contained within the cylinder;
(d) an adaptor block (7) which can be fitted to the yoke, which has formations corresponding to the formations on the gas cylinder to allow the block and the cylinder to be connected to one another; and
(e) machine readable indicia (18) associated with one of the cylinder and the block, by which the gas cylinder with its associated block can be identified by the delivery apparatus when fitted to the yoke.

2. Apparatus as claimed in claim 1, in which the formations on the block and on the cylinder comprise mating pins (16, 17) and recesses.

3. Apparatus as claimed in claim 1 or claim 2, in which the machine readable indicia (18) are optically readable.

4. Apparatus as claimed in claim 3, in which the indicia (18) comprise regions of optically reflective and absorbing materials provided in a pre-arranged pattern.

5. Apparatus as claimed in any one of claims 1 to 4, in which the block includes additional indicia for the operator of the anaesthetic delivery apparatus to identify visually the gas cylinder to which the block is or is to be connected.

6. Apparatus as claimed in any one of claims 1 to 5, which has at least one passageway extending through it for receiving a spigot on the yoke of the anaesthetic delivery apparatus.

7. Apparatus as claimed in any one of claims 1 to 6, in which the machine readable indicia are provided on the adaptor block.

8. Apparatus as claimed in claim 7, which includes at least at least one additional set of formations and indicia for a cylinder of another gas, which can be used to form a connections between the yoke of anaesthetic delivery apparatus and a cylinder containing the said other gas.

9. Apparatus as claimed in claim 8, in which the formations on the block are in the form of one of a mating set of pins and recesses, and in which the cylinder bears formations corresponding to those on the block, by which it can be mateably connected to the block.

10. Apparatus as claimed in claim 8 or claim 9, in which the block is movable between its positions for connection to the different cylinders containing the respective gases by removal from a spigot on the yoke of the apparatus, and replacement on the spigot in a different orientation.

11. Apparatus as claimed in any one of claims 7 to 10, in which the block is movable between its positions for connection to the different cylinders containing the respective gases by sliding.

12. Apparatus as claimed in any one of claims 1 to 10, which includes means for measuring the flow rate of gas supplied from the gas cylinder.

## Patentansprüche

1. Anästhetisches Verabreichungsgerät, das aufweist,
a) ein Gaszylinder-Montagejoch (1),
b) Mittel (15) zum Lesen maschinenlesbarer Markierungen,
c) einen Gaszylinder, der dem im Zylinder enthaltenen Gas entsprechende Formelemente trägt,
d) einen am Joch befestigbaren Adapterblock (7), der den Formelementen am Gaszylinder entsprechende Formelemente aufweist, um ein Verbinden von Block und Zylinder zu ermöglichen, und
e) einem der Bauteile Zylinder und Block zugeordnete maschinenlesbare Markierungen (18), mittels derer der Gaszylinder mit dem zugeordneten Block beim Befestigen am Joch von dem Verabreichungsgerät identifiziert werden kann.

2. Gerät nach Anspruch 1, wobei die Formelemente an dem Block und an dem Zylinder miteinander zusammenpassende Stifte (16, 17) und Aussparungen aufweisen.

3. Gerät nach Anspruch 1 oder 2, wobei die maschinenlesbaren Markierungen (18) optisch lesbar sind.

4. Gerät nach Anspruch 3, wobei die Markierungen (18) Bereiche aus optisch reflektierenden und absorbierenden Materialien aufweisen, die in einem vorgegebenen Muster angeordnet sind.

5. Gerät nach einem der Ansprüche 1 bis 4, wobei der Block zusätzliche Markierungen für die Bedienungsperson des anästhetischen Verabreichungsgeräts aufweist, um den Gaszylinder, mit welchem der Block verbunden ist oder zu verbinden ist, visuell identifizieren zu können.

6. Gerät nach einem der Ansprüche 1 bis 5, das mindestens einen durchgehenden Kanal zur Aufnahme eines Zapfens an dem Joch des anästhetischen Verabreichungsgeräts aufweist.

7. Gerät nach einem der Ansprüche 1 bis 6, bei welchem die maschinenlesbaren Markierungen an dem Adapterblock angebracht sind.

8. Gerät nach Anspruch 7, das mindestens eine zusätzliche Gruppe von Formelementen und Markierungen für einen Zylinder eines weiteren Gases aufweist, die zur Herstellung einer Verbindung zwischen dem Joch des anästhetischen Verabreichungsgeräts und einem das andere Gas enthaltenden Zylinder benutzbar sind.

9. Gerät nach Anspruch 8, bei welchem die Formelemente an dem Block in Form von Stiften oder damit zusammenpassender Aussparungen ausgebildet sind und der Zylinder demjenigen des Blocks eiitsprechende Formelemente trägt, mittels derer er passend mit dem Block verbindbar ist.

10. Gerät nach Anspruch 8 oder 9, wobei der Block zwischen seinen Positionen zum Anschluß der verschiedenen, die jeweiligen Gase enthaltenden Zylinder durch Abnehmen von einem Zapfen des Jochs des Geräts und Wiederaufsetzen auf den Zapfen in einer anderen Orientierung beweglich ist.

11. Gerät nach einem der Ansprüche 7 bis 10, wobei der Block zwischen seinen Positionen zum Anschluß der verschiedenen, die jeweiligen Gase enthaltenden Zylinder durch Verschieben beweglich ist.

12. Gerät nach einem der Ansprüche 1 bis 10, das Mittel zur Messung des Strömungsdurchsatzes von aus dem Gaszylinder zugeführten Gas aufweist.

## Revendications

1. Dispositif d'alimentation en anesthésique qui comprend :
(a) un étrier de montage (1) pour une bouteille de gaz ;
(b) des moyens (15) de lecture d'indices lisibles par machine ;
(c) une bouteille de gaz qui porte des éléments de détrompage en fonction du gaz contenu à l'intérieur de la bouteille ;
(d) un bloc adaptateur (7) qui peut être adapté à l'étrier et qui possède des éléments de détrompage correspondant aux éléments de détrompage présents sur la bouteille de gaz pour permettre au bloc et à la bouteille d'être raccordés l'un à l'autre ; et
(e) des indices (18) lisibles par machine associés avec l'un d'entre la bouteille et le bloc, par lesquels la bouteille de gaz avec son bloc associé peut être identifiée par le dispositif d'alimentation lorsqu'il est fixé à l'étrier.

2. Dispositif selon la Revendication 1, dans lequel les éléments de détrompage sur le bloc et sur la bouteille comportent des broches (16, 17) et des trous borgnes qui s'accouplent.

3. Dispositif selon la Revendication 1 ou 2, dans lequel les indices (18) lisibles par machine sont lisibles de manière optique.

4. Dispositif selon la Revendication 3, dans lequel les indices (18) comportent des zones de matériaux optiquement réfléchissants et absorbants disposées selon un motif préconstitué.

5. Dispositif selon l'une quelconque des Revendications 1 à 4, dans lequel le bloc comporte des indices supplémentaires pour que l'opérateur du dispositif d'alimentation en anesthésique puisse identifier visuellement la bouteille de gaz à laquelle le bloc est, ou doit être, raccordé.

6. Dispositif selon l'une quelconque des Revendications 1 à 5, qui possède au moins un passage le traversant destiné à recevoir un ergot situé sur l'étrier du dispositif d'alimentation en anesthésique.

7. Dispositif selon l'une quelconque des Revendications 1 à 6, dans lequel les indices lisibles par machine sont prévus sur le bloc adaptateur.

8. Dispositif selon la Revendication 7, qui comprend au moins un jeu complémentaire d'éléments de détrompage et d'indices pour une bouteille d'un gaz différent, qui peut être utilisé pour former une connexion entre l'étrier du dispositif d'alimentation en anesthésique et une bouteille contenant ledit gaz différent.

9. Dispositif selon la Revendication 8, dans lequel les éléments de détrompage du bloc se présentent sous la forme de l'un d'un jeu de broches et de trous borgnes s'accouplant, et dans lequel la bouteille porte des éléments de détrompage correspondant à ceux du bloc, grâce auxquels elle peut être raccordée au bloc.

10. Dispositif selon la Revendication 8 ou la Revendication 9, dans lequel le bloc est mobile entre ses positions de raccordement aux différentes bouteilles contenant les gaz respectifs par son retrait d'un ergot sur l'étrier du dispositif et son nouveau positionnement sur l'ergot avec une orientation différente.

11. Dispositif selon l'une quelconque des Revendications 7 à 10, dans lequel le bloc est mobile par coulissement entre ses positions de raccordement aux différentes bouteilles contenant les gaz respectifs.

12. Dispositif selon l'une quelconque des Revendications 1 à 10, qui comporte des moyens pour mesurer le débit de gaz fourni par la bouteille de gaz.
